# EUROPÄISCHE PATENTSCHRIFT

(11) **EP 0 463 129 B1**
(45) Veröffentlichungstag und Bekanntmachung des Hinweises auf die Patenterteilung: **12.01.1994**
(21) Anmeldenummer: 91900765.8
(22) Anmeldetag: 19.12.1990
(51) Int. Cl.: A61B 5/14

(54) **BLUTENTNAHMEVORRICHTUNG MIT BLUTBEOBACHTUNGSKAMMER**
BLOOD SAMPLING DEVICE WITH BLOOD-VIEWING CHAMBER
DISPOSITIF DE PRELEVEMENT SANGUIN AVEC COMPARTIMENT D'OBSERVATION DU SANG

(30) Priorität: 16.01.1990 DE 4000968; 11.10.1990 DE 4032274
(43) Veröffentlichungstag der Anmeldung: 02.01.1992
(73) Patentinhaber: WENDELBORN, Dieter, D-21423 Drage (DE)
(72) Erfinder: WENDELBORN, Dieter, D-21423 Drage (DE)
(74) Vertreter: Schaefer, Konrad, Dipl.-Phys.
(86) Internationale Anmeldenummer: EP9002243
(87) Internationale Veröffentlichungsnummer: WO9110397

(56) Entgegenhaltungen:
- WO-A-88/02238
- DE-A- 2 835 101
- GB-A- 1 345 979
- US-A- 3 931 815
- US-A- 4 821 738

## Beschreibung

Die Erfindung betrifft eine Blutentnahmevorrichtung.

Derartige mittels Vakuum ansaugende Blutentnahmevorrich tungen ersetzen in letzter Zeit zunehmend die herkömmlich für diese Zwecke verwendeten Kolbenspritzen. Bei der Ansaugung des Blutes in ein evakuiertes Entnahmegefäß kann der Saugdruck aber nicht fein reguliert werden. Wenn die Kanüle den Stopfen durchstochen hat, wird mit vollem Vakuumdruck gesaugt. Dies hat für andere Zwecke Vorteile, ist aber nachteilig dann, wenn die Kanüle die Vene nicht getroffen hat, sondern im danebenliegenden Gewebe liegt. Unter der vollen Saugkraft des Vakuums wird dann im Gewebe ein Hämatom erzeugt, das für den Patienten unangenehm ist. Außerdem ist ein evakuiertes Entnahmegefäß eröffnet und belüftet worden, ohne Blut anzusaugen und muß beim nächsten Versuch, eine Vene zu punktieren, durch ein neues Entnahmegefäß ersetzt werden.

Blutentnahmevorrichtungen der eingangs genannten Art weisen daher eine Blutbeobachtungskammer auf, die außerhalb des Entnahmegefäßes vorgesehen ist und es gestattet, das aus der Vene ausströmende Blut zu erkennen, bevor Vakuum an die Kanüle gelegt wird, bevor also mit der Kanüle der Stopfen durchstochen wird. Die Ausbildung der Blutbeobachtungseinrichtung als Kammer ist angesichts der heute durch Aids, Hepatitis etc. stark erhöhten Hygieneanforderungen erforderlich, um Berührungen des Bedienungspersonals mit Patientenblut vollständig zu verhindern.

Mit einer solchen Blutentnahmevorrichtung kann unter Umständen mehrfach suchend ins Gewebe gestochen werden, bis die gesuchte Vene punktiert ist. Dies wird daran festgestellt, daß Blut in die Blutbeobachtungskammer eintritt. Dann wird die Kanüle durch den Stopfen gestochen.

Eine Blutentnahmevorrichtung der eingangs genannten Art ist aus dem Stand der Technik bekannt, in Form der DE-PS 28 35 101. Bei dieser bekannten Konstruktion ist die Blutbeobachtungskammer am mittleren Bereich der Kanüle vorgesehen. Zu diesem Zweck ist die Kanüle zweiteilig ausgebildet. Im Gegensatz zu anderen bekannten Konstruktionen dieser Art ist bei dieser Konstruktion vorgesehen, daß das Blut in die Beobachtungskammer angesaugt wird. Zu diesem Zweck wird die Kanüle durch den Stopfen hindurch in eine über eine bewegliche Membran vakuumbeaufschlagte Saugkammer gestoßen, die mit auf diese Weise konstruktiv begrenzter Saugwirkung das Blut in die Blutbeobachtungskammer saugt.

Nachteilig bei dieser bekannten Konstruktion sind zum einen die Herstellungskosten aufgrund der komplizierten Kanülenkonstruktion. Aber auch bei der Bedienung der bekannten Blutentnahmevorrichtungen durch den Arzt ergeben sich wesentliche Nachteile, die dadurch bedingt sind, daß die Blutbeobachtungskammer an der Kanüle vorgesehen ist. Meistens ist es bei der Blutentnahme erforderlich, mehrere Entnahmegefäße nacheinander zu füllen, die für unterschiedliche Blutuntersuchungen benötigt werden. Man läßt dabei die Kanüle in der Vene des Patienten liegen und setzt nacheinander die Entnahmegefäße an. Beim Wechsel des Entnahmegefäßes bleibt aber Blut in der Blutbeobachtungskammer für einige Zeit stehen und beginnt den Blutgerinnungsprozeß.

Dies kann zu Verstopfungen führen, so daß auch die Vakuumwirkung des nächsten angesetzten Entnahmegefäßes nicht mehr ausreicht, um Blut anzusaugen. Ferner kann das teilweise geronnene Blut aus der Blutbeobachtungskammer in dem nächsten angesetzten Entnahmegefäß das dort üblicherweise vorgesehene Antikoagulanz unwirksam machen, was zu Verfälschungen der Untersuchungsergebnisse führt.

Aus diesen Gründen konnte sich der genannte Stand der Technik bisher in der Praxis nicht durchsetzen.

Die Aufgabe der vorliegenden Erfindung besteht daher darin, eine Blutentnahmevorrichtung der eingangs genannten Art zu schaffen, die unter Vermeidung von Koagulationsproblemen im Kanülenbereich konstruktiv vereinfacht und verbilligt ist.

Diese Aufgabe wird erfindungsgemäß mit den Merkmalen des Kennzeichnungsteils des Anspruches 1 gelöst.

Die erfindungsgemäße Blutentnahmevorrichtung sieht die Kammer am Stopfen vor und nicht an der Kanüle. Diese Kammer ist in Stichrichtung dem Stopfen vorgeschaltet und wird bei Einstichbewegung zunächst angestochen. Liegt die Kanüle mit ihrem vorderen Ende in einer Vene, so kann Blut in die Kammer austreten und durch deren durchsichtige Seitenwände hindurch erkannt werden. Dann kann der Stopfen durchstochen und Blut entnommen werden. Anschließend wird die Kanüle aus dem Stopfen und der Membran herausgezogen. Das Blut in der Kammer wird zusammen mit dem ohnehin mit Blut in Berührung stehendem Stopfen entsorgt. Da an der Kanüle keine Kammer mehr vorgesehen ist, werden bei Mehrfachblutentnahme mit einer Kanüle die beschriebenen Koagulationsprobleme vermieden. Von Vorteil ist auch die Möglichkeit, die Kanüle und den daran gegebenenfalls vorgesehenen Halter unverändert zu lassen. Zur Ausbildung der vorliegenden Erfindung muß lediglich am Stopfen eine geringfügige Änderung vorgenommen werden, was mit erheblich geringeren Umrüstkosten verbunden ist.

Vorteilhaft sind die Merkmale des Anspruches 2 vorgesehen. Die Stopfen der Blutentnahmegefäße sind in der Regel mit derartigen Schutzkappen ausgerüstet, die verhindern sollen, daß der Laborant beim Öffnen des Entnahmegefäßes im Labor mit der blutigen Unterseite des Stopfens in Berührung kommt. Die Schutzkappe besteht ohnehin in der Regel aus anderem Material als der Stopfen und kann daher leichter zur Ausbildung der Kammer geformt und insbesondere leichter aus durchsichtigem Material gestaltet werden, was beim Stopfen selbst wegen der geforderten Elastizitäts- und Vakuumfestigkeitseigenschaften nur schwer möglich ist.

Weiterhin vorteilhaft sind die Merkmale des Anspruches 3 vorgesehen. An der Kanüle ist bei handelsüblichen Blutentnahmevorrichtungen in der Regel ein solcher Halter vorgesehen, der rohrförmig den Stopfen und das Blutentnahmegefäß umschließt. Zum Durchstoßen des Stopfens wird daher der Stopfen bzw. die am Stopfen vorgesehene Schutzkappe im rohrförmigen Halter bewegt. Mit Anschlageinrichtungen kann dafür gesorgt werden, daß beim Einstechen eine Stellung leicht gefunden und präzise eingehalten wird, bei der das hintere Kanülenende in der Kammer steht. Durch den Anschlag wird also sichergestellt, daß die Kanüle in der korrekten Beobachtungsstellung steht.

Weiterhin vorteilhaft sind die Merkmale des Anspruches 4 vorgesehen. Die Membran muß selbstverständlich flüssigkeitsdicht sein, um in der Kammer enthaltenes Blut zurückzuhalten. Sie ist luftdurchlässig, um mit ihrem Innendruck stets im Druckausgleich mit der Umgebung zu stehen. Dadurch wird insbesondere vermieden, daß in der Kammer sich ein Überdruck einstellt, der beispielsweise durch Herstellungsfehler oder beim Gebrauch in größerer Höhe (z.B. Flugzeug) auftreten kann. Überdruck in der Kammer könnte das Eintreten von Blut in die Kammer, also die korrekte Anzeige verhindern.

Weiterhin vorteilhaft sind die Merkmale des Anspruches 5 vorgesehen. Eine solche enge Entlüftungsöffnung in der Wand der Kammer sorgt für wirkungsvollen Druckausgleich des Kammerinneren gegenüber der umgebenden Atmosphäre, so daß Blut aus der Kanüle stets ungehindert in die Kammer eintreten kann. Die Öffnung ist so eng, daß sie kein Blut passieren läßt. Dafür sorgt die Oberflächenspannung des Blutes gegenüber dem hydrophoben Wandmaterial. Solange die bei sachgerechter Handhabung auftretenden Druck- und Krafteinflüsse nicht überschritten werden, sperrt die Öffnung jeden Blutdurchtritt.

Weiterhin vorteilhaft sind die Merkmale des Anspruches 6 vorgesehen. Diese Art der Anschlageinrichtung ist je nach Verdrehwinkel zwischen Halter und Stopfen in bzw. außer Anschlageingriff bringbar. Wird in bestimmter, beispielsweise durch Markierungen vorgegebener Winkelstellung vorgeschoben, so erfolgt Anschlag. Nach Drehung in eine andere markierte Winkelstellung gelangen der Innenvorsprung und die Längsnut in Eingriff, so das zum Durchstechen des Stopfens weiter vorgeschoben werden kann.

Weiterhin vorteilhaft sind die Merkmale des Anspruches 7 vorgesehen. Auf diese Weise wird die Membran im radial innenliegenden, zum Durchstechen durch die Kanüle bestimmten mittleren Bereich dünn und somit leicht durchstechbar ausgebildet. Dieser dünne, nachgiebige Bereich wird aber durch den außen anschließenden dickeren Bereich der Membran soweit stabilisiert, daß er auch von einer stumpfen Kanüle ohne größere Auslenkung durchstochen wird.

In den Zeichnungen ist die Erfindung beispielsweise und schematisch dargestellt. Es zeigen:
- Fig. 1: eine Achsschnittdarstellung einer ersten stark schematisierten Ausführungsform der Erfindung,
- Fig. 2: einen Explosionsschnitt durch ein Blutentnahmegefäß einer zweiten Ausführungsform,
- Fig. 3: eine perspektivische Ansicht der Vakuumblutentnahmevorrichtung gemäß der zweiten Ausführungsform der Erfindung,
- Fig. 4: eine perspektivische Ansicht der Vakuumblutentnahmevorrichtung gemäß einer dritten Ausführungsform der Erfindung,
- Fig. 5 und 6: Seitenansichten der Ausführungsform der Figur 3 in den beiden wesentlichen Betriebsstellungen.

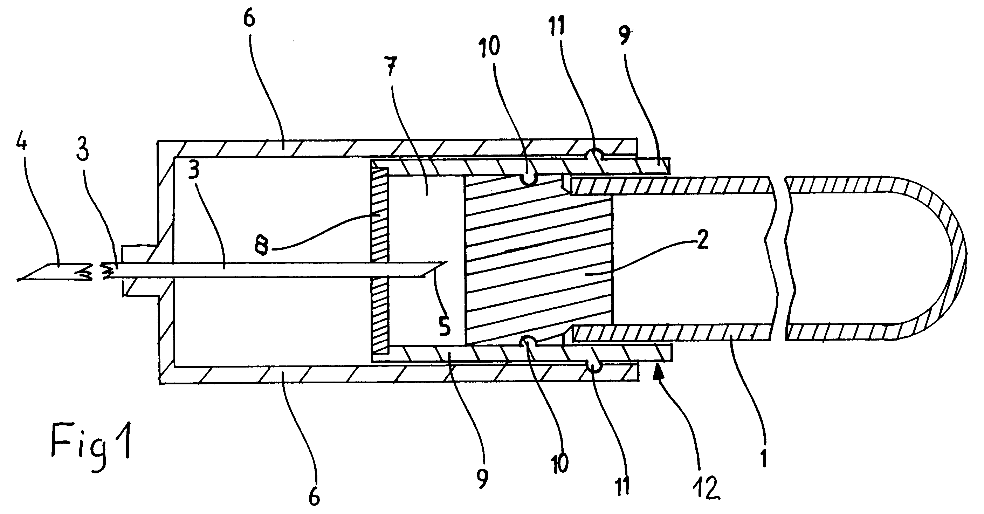

Figur 1 zeigt die Erfindung in einem stark schematisierten Ausführungsbeispiel.

Im wesentlichen besteht eine Blutentnahmevorrichung der hier fraglichen Art aus einem Entnahmegefäß 1, einem Stopfen 2 und einer Kanüle 3.

Das Entnahmegefäß 1 ist in der Regel, wie in der Zeichnung dargestellt, in der üblichen Reagenzglasform ausgebildet und besteht aus geeignet vakuumfestem und vorteilhafter Weise durchsichtigem Material, wie beispielsweise Glas oder Kunststoff. Das Innere des Entnahmegefäßes 1 ist evakuiert. Das offene Ende des Entnahmegefäßes ist mit einem Stopfen 2 verschlossen, der aus geeignet vakuumdichtem elastischem Material besteht, das zudem selbstschließend ausgebildet ist, einen von einer Kanüle durchstochenen Stichkanal nach Herausziehen der Kanüle also wieder schließt, zumindest flüssigkeitsdicht schließt.

Die Kanüle 3 ist an beiden Enden zugespitzt, und zwar am vorderen, dem Patienten zugewandten Ende 4 und auch am hinteren, dem Stopfen 2 zugewandten Ende 5. Mit dem vorderen Ende 4 wird die Kanüle 3 in die Vene eines Patienten gestochen. Das hintere Ende 5 wird durch den Stopfen 2 ins Innere des Entnahmegefäßes 1 gestochen. Das Vakuum im Inneren des Entnahmegefäßes 1 saugt sodann Blut durch die Kanüle aus der Vene des Patienten an und füllt das Entnahmegefäß 1. Die Kanüle 3 wird dann aus dem Stopfen 2 herausgezogen, und das mit dem Stopfen 2 verschlossene Entnahmegefäß 1 kann nun in ein Labor gebracht werden, wo nach Abziehen des Stopfens 2 das Blut untersucht werden kann.

Üblicherweise ist an der Kanüle 3 ein Halter 6 vorgesehen, der mit einer vorderen Querwand und mit einem verdickten Nabenbereich am mittleren Teil der Kanüle 3 befestigt ist und rohrförmig den Stopfen 2 und das Entnahmegefäß 1 übergreift. Der Halter 6 erleichtert das Manipulieren der Kanüle beim Einstechen in die Vene des Patienten und erleichtert mit seiner rohrförmig führenden Umschließung des Stopfens 2 das korrekt zentrierte Einstechen der Kanüle in den Stopfen 2.

Um erkennen zu können, ob beim Einstechen der Kanüle 3 in den Patienten eine Vene getroffen ist, muß das hintere Ende 5 beobachtet werden. Tritt dort Blut aus, so ist eine Vene getroffen. Tritt kein Blut aus, so muß die Kanüle ein Stück zurückgezogen und erneut vorgestochen werden. Dieses nur zur Anzeige dienende Blut soll nicht in die Umgebung gelangen, sondern in der Blutentnahmevorrichung zurückgehalten werden. Es ist daher eine Kammer 7 vorzusehen, in der das Blut gut zu sehen ist, aber umschlossen bleibt.

Erfindungsgemäß ist diese Kammer 7 auf der dem Entnahmegefäß abgewandten Seite des Stopfens 2 vorgesehen. Die Kammer wird, in Richtung der Kanüle 3 gesehen, vom Stopfen 2 und von einer vor diesem angeordneten Membran 8 begrenzt und seitlich von einer aus einer zylindrischen Rohrwand 9 bestehenden Schutzkappe 12 aus durchsichtigem Material, die dichtend den Stopfen 2 umschließt. Die Kammer 7 ist auf diese Weise allseitig abgedichtet umschlossen. Seitlich durch die Rohrwand 9 kann das hintere Ende 5 der Kanüle 3 und dort gegebenenfalls austretendes Blut beobachtet werden, wenn die Kanüle in der in der Figur dargestellten Stellung steht. Wird Blutaustritt beobachtet, so ist dies eine Anzeige dafür, daß die Kanüle mit ihrem vorderen Ende 4 in einer Vene steht. Das Entnahmegefäß 1 kann sodann weiter in Richtung auf die Kanüle vorgeschoben werden, so daß das hintere Ende 5 der Kanüle den Stopfen 2 durchstößt.

Im dargestellten Ausführungsbeispiel verbindet die Rohrwand 9 nicht nur die Membran 8 und den Stopfen 2, sondern überragt den Stopfen bis über den Halsbereich des Entnahmegefäßes 1. Die Rohrwand 9 dient also hier als die üblicherweise ohnehin vorgesehene Schutzkappe 12, die verhindern soll, daß im Labor beim Abziehen des Stopfens der Laborant mit seinen Fingern Berührung mit der blutigen Unterseite des Stopfens 2 bekommt.

Im dargestellten Ausführungsbeispiel ist der Stopfen 2 in der Innenseite der Rohrwand 9 mit Vorsprüngen 10 arretiert. Es können auch andere geeignete Halterungsmöglichkeiten für den Stopfen vorgesehen sein.

Die Rohrwand 9 besitzt auf der Außenseite Rastnocken 11, die in der dargestellten Längsstellung in Eingriff mit Rastnuten auf der Innenseite des Halters 6 gelangen und auf diese Weise eine Anschlageinrichtung bilden, die beim Vorschub des Stopfens 2 in Richtung auf die Kanüle 3 eine Stellung festlegt, bei der das hintere Ende 5 der Kanüle 3 genau in der Kammer 7 steht. Mit etwas erhöhter Vorschubkraft kann der Anschlag überwunden und der Stopfen 2 weiter vorgeschoben werden.

Beim dargestellten Ausführungsbeispiel, bei dem an der Kanüle 3 ein Halter 6 vorgesehen ist, der rohrförmig die Kammer 7 umgibt, muß das Blut in der Kammer 7 durch zwei Wände 6, 9 hindurch beobachtet werden. Es ist daher dafür Sorge zu tragen, daß die Materialien dieser Wände geeignet durchsichtig sind.

Das Material der Membran 8 soll von der Kanüle gut durchstechbar und selbfitschließend sein, ebenso wie der Stopfen 2, kann in der Regel also aus demselben Material vorgesehen sein. Bei der Konstruktion der Membran ist auch dafür Sorge zu tragen, daß ein Überdruck in der Kammer vermieden wird. Dies kann durch besonders flexible, beispielsweise balgförmige Ausbildung der Membran erreicht werden oder durch Ausbildung der Membran aus einem Material, das wasserundurchlässig, aber luftdurchlässig ist und auf diese Weise stets für Gleichdruck der Kammer mit der Umgebung sorgt. Ferner kann auch ein sehr kleines Lüftungsloch in der Rohrwand 9 vorgesehen sein.

In vereinfachter Ausführungsform kann die Kammer 7 auch vollständig vom Material des Stopfens 2 umschlossen, also als Hohlraum im Stopfen ausgebildet sein, was die Konstruktion vereinfacht, aber wegen der erforderlichen Durchsichtigkeit zu Materialproblemen führen kann.

Die Kammer 7 kann auch, abweichend vom dargestellten Ausführungsbeispiel andere geometrische Formen nehmen, beispielsweise auf der Außenseite des Stopfens 2 als halbkugelförmige Membranblase aus durchsichtigem Material ausgebildet sein. In der Regel wird es aber vorteilhafter sein, wie beim dargestellten Ausführungsbeispiel mit der Rohrwand 9 die Seitenwand aus gesondertem, gut durchsichtigem Material herzustellen. Außerdem besteht die Möglichkeit, die Kammer so auszubilden, daß sie gegenüber dem Stopfen 2 noch eine zusätzliche Trennwand aufweist, was Herstellungsvorteile mit sich bringen kann.

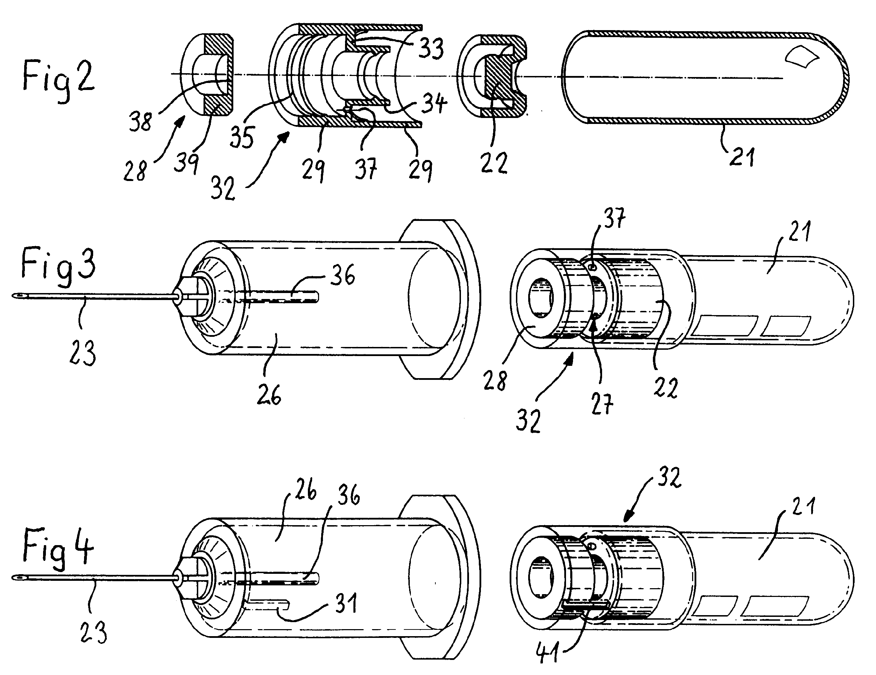

Die Figuren 2, 3, 5 und 6 zeigen eine bevorzugte Ausführungsform der Erfindung, die in den Grundzügen mit der vereinfachten Ausführungsform der Figur 1 übereinstimmt . Es werden, wo möglich, dieselben Bezugszeichen verwendet, jeweils um die Zahl 20 erhöht.

Figur 2 zeigt, daß das Entnahmegefäß 21 der üblichen Grundform entspricht. Die Schutzkappe 32 ist aber wesentlich verändert, und zwar insbesondere spritzgußtechnischen Erfordernissen angepaßt. Ihre Rohrwand 29 weist in der Längsmitte einen Innenflansch 33 auf, der einen zum Entnahmegefäß 21 hin weisenden Rohrstutzen 34 trägt, auf dem in üblicher Bauweise der Stopfen 22, der ebenfalls üblicher Bauweise entspricht, aufgesteckt und sicher gehalten ist.

Auf der dem Entnahmegefäß 21 abgewandten Seite des Innenflansches 33 ist die Rohrwand 29 mit umlaufenden Innenrippen 35 versehen. Diese dienen zum Halten der Membran 28, die wie ein Stopfen bündig in das Ende der Rohrwand 29 eingeschoben wird, um an den Innenrippen 35 einen Begrenzungsanschlag zu finden.

Die Membran 28 weist einen radial innenliegenden Bereich 38 geringerer Wandstärke und einen radial außenliegenden Bereich 39 größerer Wandstärke auf. Hierdurch wird erreicht, daß der innere dünnere Bereich 38 zwar dünn und somit von der Kanüle 3 leicht durchstechbar ist, dennoch aber von dem dickeren außenliegenden Bereich 39 so gut stabilisiert ist, das er unter dem Druck der einstechenden Kanüle 3 nur wenig nachgibt, so daß ein Durchstechen auf kurzem Wege sichergestellt ist.

Zum Druckausgleich im Inneren der Kammer 27 kann die Membran 28 aus gasdurchlässigem Material gestaltet werden. Vorzuziehen ist aber die Anordnung einer Entlüftungsöffnung in Form einer Bohrung 37, die von der Kammer 27 her den Innenflansch 33 durchsetzt. Das Material, in dem die Bohrung 37 vorgesehen ist, muß hydrophob ausgebildet sein. Bei vorgegebener Oberflächenspannung des Blutes und bei vorgegebenen maximalen das Blut durch die Bohrung pressenden Kräften, die aufgrund von Druckunterschieden oder Beschleunigungskräften entstehen können, wie sie bei sachgemäßer Behandlung nicht überschritten werden, kann unterhalb eines bestimmten Bohrungsdurchmessers aufgrund der Oberflächenspannung Blut nicht mehr durch die Bohrung treten.

Die Bohrung 37 verbindet also die Kammer 27 mit dem ringförmigen Bereich zwischen der Rohrwand 29 und dem den Stopfen 22 aufnehmenden Rohrstutzen 34. Je nach Ausbildung des Stopfens 22 kann es vorkommen, daß dieser beim Aufsetzen stirnseitig gegen den Innenflansch 33 gedrückt wird und die Bohrung 37 verschließt. Dann wäre die ordnungsgemäße Belüftung der Kammer 27 verhindert.

Um dies zu vermeiden, ist vorteilhaft im Bereich um die Bohrung 37 herum der Innenflansch 33 durch eine Vertiefung auf seiner dem Stopfen 22 zugewandten Seite etwas dünner ausgebildet, wie in Figur 2 der Schnitt durch den Innenflansch 33 zeigt. Im Bereich der Bohrung 37 kann der Stopfen 22 also nicht auf der Öffnung der Bohrung 37 aufsetzen.

Dadurch wird die Entlüftungsmöglichkeit immer freigehalten.

Figur 3 zeigt in perspektivischer Ansicht das Entnahmegefäß 21 mit komplett montierter zusammengesetzter und auf das Entnahmegefäß gesteckter Schutzkappe 32. Es sind zu erkennen die Membran 28 mit ihrer charakteristischen Formgebung, die in Figur 2 dargestellt ist, der Stopfen 22 in der Rohrwand 29 und auch die Bohrung 37. Es ist aus dieser Darstellung ersichtlich, daß die Schutzkappe mit der Rohrwand 29 aus durchsichtigem Material besteht, das das Innere der Kammer 27 einsehen läßt.

In Figur 3 ist auch ein Halter 26 mit Kanüle 23 dargestellt, der im wesentlichen der Ausführungsform der Figur 1 entspricht. Der innere Teil der Kanüle 33 ist nicht zu sehen, da er von dem üblichen geschlossenen Gummischlauch 36 verdeckt ist, der beim Durchstoßen der Membran 28 oder des Stopfens 22 vom hinteren Ende 25 der Kanüle 23 mit durchstochen wird und nach Herausziehen der Kanüle wieder seine alte Form annimmt und die Kanüle blutdicht verschließt.

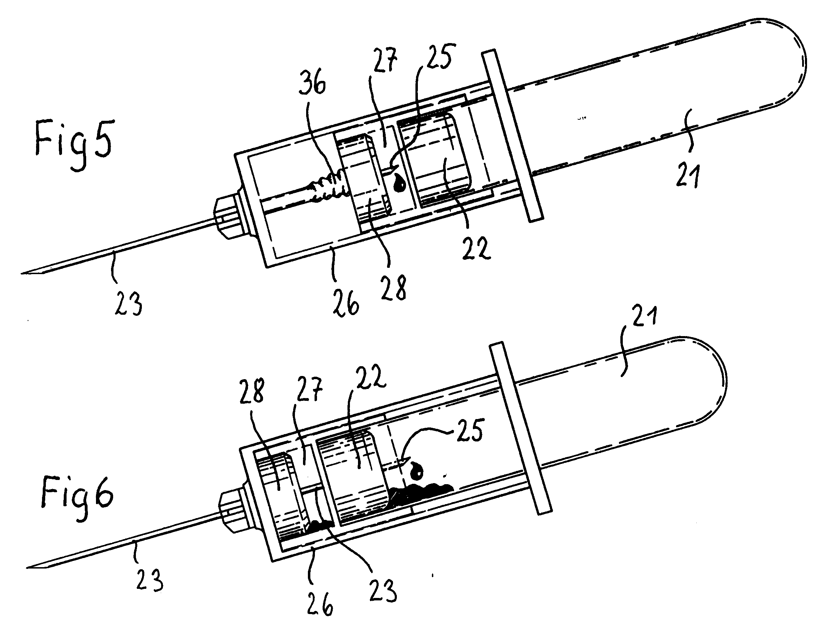

Figur 5 zeigt die Ausführungsform der Figur 3 in Seitenansicht, wobei das komplett montierte mit Stopfen 22 versehene Entnahmegefäß 21 so weit auf die Kanüle 23 gestochen ist, daß deren hinteres Ende 25 unmittelbar in der Kammer 27 steht. Es ist ein austretender Blutstropfen dargestellt. Es ist hier auch zu ersehen, wie sich der Gummischlauch 36 beim Durchstechen der Membran 28 auf der Kanüle 23 zusammenstaucht.

Figur 6 zeigt in Darstellung der Figur 5 die Betriebsstellung, bei der das Entnahmegefäß 1 bis zum Anschlag auf die Kanüle 23 geschoben ist, so daß das hintere Ende 25 der KanÄle nunmehr im Inneren des Entnahmegefäßes 21 steht, so daß dort Blut (wie dargestellt) vom Vakuum angesaugt werden kann.

Der Vergleich der Figuren 5 und 6 zeigt, daß im wesentlichen zwei Vorschubstellungen des Entnahmegefäßes 21 gegenüber der Kanüle 23 bzw. dem Halter 26 erforderlich sind, wobei die Vorschubstellung der Figur 6 durch natürlichen Anschlag am Grund des Halters 26 von selbst eingestellt wird, die Stellung der Figur 5 aber vom Arzt mit Fingerspitzengefühl und unter Beobachtung einzustellen ist.

Dies kann erleichtert werden durch die Anschlageinrichtung 11 der Ausführungsform der Figur 1. Diese hat allerdings den Nachteil, daß sie unter Umständen schwergängig sein kann und unter Kraftaufwendung zum Verwackeln und somit zur Schmerzbeeinflussung des Patienten führt.

Bei der Ausführungsform der Figur 4, die derjenigen der Figur 3 in allen sonstigen Teilen entspricht, ist an der Innenseite der Zylinderwand des Halters 26 ein nach innen ragender, in Achsrichtung erstreckter Innenvorsprung 31 vorgesehen. In der Rohrwand 29 der Schutzkappe 32 ist von der Außenseite her eine in Längsrichtung erstreckte Längsut 41 vorgesehen, die den Innenvorsprung 31 aufnehmen kann, wenn die in Figur 4 dargestellte Winkelstellung der Teile zueinander eingehalten wird. Dann kann das Entnahmegefäß 21 bis zum Grund des Halters 26 vorgeschoben werden, bis also die Betriebsstellung der Figur 6 erreicht ist. Sind die beiden in Figur 4 dargestellten Teile jedoch gegeneinander verdreht, so kann nur bis zum Anschlag der Stirnfläche der Rohrwand 29 gegen den Innenvorsprung 31 vorgeschoben werden, was der Stellung der Figur 5 entspricht. Bei dieser Ausführungsform kann also mit leichter Hand bis zum Anschlag vorgeschoben und sodann nach Verdrehen feinfühlig weiter vorgeschoben werden.

In Fällen, in denen das Entnahmegefäß 21 und die Schutzkappe 32 im Durchmesser sehr klein sind in bezug auf den Innendurchmesser des Halters 26, kann es sich empfehlen, auf der Außenseite der Rohrwand 29 flanschartige Verbreiterungen zur Führung im Halter 26 vorzusehen. Auf diesen kann dann die Nut 41 vorgesehen sein.

Die in Figur 2 im Schnitt dargestellte Schutzkappe 32 ist auf spritzgußtechnische Herstellbarkeit optimiert. Sie kann, wie der Fachmann sofort sieht, mit aus beiden Richtungen in Achsrichtung zusammengesetzten Kernen in einer Form gespritzt werden. Dies gilt insbesondere auch für die Anordnung der Bohrung 37, die in Achsrichtung liegt und somit in der Entformungsrichtung der Kerne entformbar ist.

Auch bei der Gestaltung der Anschlageinrichtung bei der Ausführungsform der Figur 4 ist auf spritzgußtechnische Herstellbarkeit optimiert worden Die Längsnut 41 ist in Längsrichtung angeordnet, um auch die Nut in Entformungsrichtung der Kerne, also in Achsrichtung entformbar zu machen, so daß die Form einfach und kostengünstig gehalten werden kann. Dies gilt auch für den Innenvorsprung 31 am Halter 26, der in Achsrichtung angeordnet ist und daher auf gleiche Weise für einfache Entformbarkeit bei einfacher Formkonstruktion sorgt.

## Patentansprüche

1. Blutentnahmevorrichtung mit einer an beiden Enden zugespitzten Kanüle (3), einem vorevakuierten Entnahmegefäß (1) mit einem selbstschließend durchstechbaren Stopfen (2), einer selbstschließend durchstechbaren Membran (8) und einer in Stichrichtung vor dem Stopfen angeordneten, zu den Seiten hin von durchsichtigem Material umschlossenen Blutbeobachtungskammer (7,27), dadurch gekennzeichnet, daß die Kammer (7, 27) am Stopfen (2, 22) angeordnet ist, wobei sie zum Entnahmegefäß (1) hin im wesentlichen vom Stopfen (2, 22) und zum Patienten hin von der Membran (8, 28) umschlossen ist.

2. Blutentnahmevorrichtung nach Anspruch 1 mit einer rohrförmig den Hals des Entnahmegefäßes überfassenden, am Stopfen befestigten Schutzkappe, dadurch gekennzeichnet, daß die Schutzkappe (12, 32) aus durchsichtigem Material (9, 29) besteht, den Stopfen (2, 22) zum Patienten hin überragt und an ihrem dortigen Ende die Membran (8, 28) trägt.

3. Blutentnahmevorrichtung nach einem der vorhergehenden Ansprüche mit einem im mittleren Bereich der Kanüle befestigten, rohrförmig den Stopfen umgreifenden Halter, dadurch gekennzeichnet, daß am Halter (6, 26) und am Stopfen (2, 22) bzw. an der Schutzkappe (12, 32) Anschlageinrichtungen (11, 31) vorgesehen sind, die bei Stichbewegung in Anschlag gelangen, wenn das hintere Ende (5) der Kanüle (3) in der Kammer (7, 27) steht.

4. Blutentnahmevorrichtung nach einem der vorhergehenden Ansprüche, dadurch gekennzeichnet, daß die Membran (8, 28) aus luftdurchlässigem, flüssigkeitsundurchlässigem Material besteht.

5. Blutennahmevorrichtung nach einem der vorhergehenden Ansprüche, dadurch gekennzeichnet, daß die Kammer (7, 27) eine Entlüftungsöffnung aufweist, die aus einer Bohrung (37) in einer Wand (33) aus hydrophobem Material besteht, deren Durchmesser so klein ist, daß die Entlüftungsöffnung unter bei sachgerechter Handhabung auftretenden Druck- und Krafteinflüssen kein Blut hindurchtreten läßt.

6. Blutentnahmevorrichtung nach Anspruch 3, dadurch gekennzeichnet, daß die Anschlageinrichtung einen achsparellelen Innenvorsprung (31) am nadelseitigen Ende des Halters (26) und eine Längsnut (41) auf der Außenfläche des Stopfens (22) bzw. der Schutzkappe (32) aufweist.

7. Blutentnahmevorrichtung nach einem der vorhergehenden Ansprüche, dadurch gekennzeichnet, daß die Membran (28) einen radial innenliegenden Bereich (38) geringerer Wandstärke aufweist.

## Claims

1. Blood sampling device with a cannula (3), which is pointed at both ends, a pre-evacuated sampling vessel (1) with a self-closing pierceable plug (2), a self-closing pierceable membrane (8) and a blood viewing chamber (7,27) which is arranged in front of the plug in the piercing direction and is enclosed at the sides by transparent material, characterised in that the chamber (7,27) is arranged at the plug (2,22), whereby it is enclosed towards the sampling vessel (1) substantially by the plug (2, 22) and towards the patient by the membrane (8,28).

2. Blood sampling device as claimed in claim 1 with a tubular protective cap which engages around the throat of the sampling vessel and is secured to the plug, characterised in that the protective cap (12,32) comprises transparent material (9,29), extends beyond the plug (2,22) towards the patient and at that end carries the membrane (8,28).

3. Blood sampling device as claimed in one of the preceding claims with a tubular holder which extends around the plug and is connected in the central region of the cannula, characterised in that provided on the holder (6,26) and on the plug (2,22) or on the protective cap (12,32) are abutment devices (11,31) which come into abutment when piercing movement occurs when the rear end (5) of the cannula (3) is situated within the chamber (7,27).

4. Blood sampling device as claimed in one of the preceding claims, characterised in that the membrane (8,28) comprises air-permeable, liquid-impermeable material.

5. Blood sampling device as claimed in one of the preceding claims, characterised in that the chamber (7,27) has a vent opening which comprises a bore (37) in a wall (33) of hydrophobic material, the diameter of which is so small that the vent opening permits no blood to flow through it under the effects of pressure and force which occur, when properly used.

6. Blood sampling device as claimed in claim 3, characterised in that the abutment device has an internal projection (31) parallel to the axis at the needle end of the holder (26) and a longitudinal groove (41) on the outer surface of the plug (22) or the protective cap (32).

7. Blood sampling device as claimed in one of the preceding claims, characterised in that the membrane (28) has a radially inner region (38) of smaller wall thickness.

## Revendications

1. Dispositif de prélèvement sanguin muni d'une canule (3) dont les deux extrémités sont en pointe, d'un récipient de prélèvement préalablement évacué (1) et muni d'un bouchon (2) pouvant être transpercé et se refermant de lui-même, d'une membrane pouvant être transpercée et se refermant d'elle-même (8), ainsi que d'un compartiment d'observation du sang (7, 27) disposé en amont du bouchon dans le sens de la percée et dont le côté est entouré d'un matériau transparent, **caractérisé en ce que** le compartiment (7, 27) est placé devant le bouchon (2, 22) et fermé essentiellement par le bouchon (2, 22) du côté du récipient de prélèvement et par la membrane (8, 28) du côté du patient.

2. Dispositif de prélèvement sanguin selon la revendication 1 muni d'un capuchon de protection fixé au bouchon et entourant de façon tubulaire le col du récipient de prélèvement, **caractérisé en** **ce que** le capuchon de protection (12, 32) est constitué d'un matériau transparent (9, 29) dépassant le bouchon (2,22) du côté du patient et portant la membrane (8, 28) à cette extrémité.

3. Dispositif de prélèvement sanguin selon l'une des revendications précédentes muni d'un manchon fixé au niveau de la zone médiane de la canule et entourant de façon tubulaire le bouchon, **caractérisé en ce que** le manchon (6, 26), le bouchon (2, 22) et le capuchon de protection (12, 32) sont munis de dispositifs de butée (11, 31) assurant un arrêt lorsque l'extrémité arrière (5) de la canule (3) se trouve dans le compartiment (7, 27).

4. Dispositif de prélèvement sanguin selon l'une des revendications précédentes **caractérisé en ce que** la membrane (8, 28) est constituée d'un matériau perméable à l'air et imperméable aux liquides.

5. Dispositif de prélèvement sanguin selon l'une des revendications précédentes **caractérisé en ce que** le compartiment (7, 27) est muni d'un orifice de purge d'air sous forme d'un orifice (37) dans une paroi (33) en matériau hydrophobe, le diamètre de l'orifice étant assez petit pour ne pas laisser passer de sang sous l'action des forces et pressions excercées lors de la manipulation correcte du dispositif.

6. Dispositif de prélèvement sanguin selon la revendication 3, **caractérisé en ce que** le dispositif de butée est constitué d'une languette intérieure (31) parallèle à l'axe du dispositif de prélèvement située du côté canule du manchon (26) et d'une rainure longitudinale (41) sur la face extérieure du bouchon (22) et du capuchon de protection (32).

7. Dispositif de prélèvement sanguin selon l'une des revendications précédentes **caractérisé en ce que** la partie radiale intérieure (38) de la membrane (28) est d'épaisseur réduite
